# EUROPEAN PATENT APPLICATION

(11) **EP 4 666 984 A1**
(43) Date of publication of application: **24.12.2025**
(21) Application number: 23923060.0
(22) Date of filing: 08.12.2023
(51) Int. Cl.: A61C 8/00, A61C 3/02, A61B 17/16

(54) **DRILL TOOL FOR LIFTING SINUS**

(30) Priority: 13.02.2023 KR 20230018766
(71) Applicant: Megagen Implant Co., Ltd., Dalseong-gun, Daegu 42921 (KR)
(72) Inventor: PARK, Kwang Bum, Daegu 42016 (KR); AN, Hyun Wook, Daegu 42677 (KR)
(74) Representative: Patentanwälte Hemmer Lindfeld Frese Partnerschaft mbB
(86) International application number: PCT/KR2023/020191
(87) International publication number: WO 2024/172261

(57) **Abstract**

Disclosed is a drill tool for maxillary sinus augmentation. A drill tool for maxillary sinus augmentation according to an embodiment of the present inventive concept includes: an implant drill including a drill hole diameter expansion edge, whereby damage to a membrane of a maxillary sinus is prevented and a diameter of a drill hole is expanded when a maxillary sinus augmentation procedure is performed; and a drill housing that is detachably coupled to the implant drill and guides a drilling operation by the implant drill.

## Description

### TECHNICAL FIELD

The present inventive concept relates to a drill tool for maxillary sinus augmentation, and more particularly, to a drill tool for maxillary sinus augmentation that may easily expand the diameter of a drill hole while preventing damage to a maxillary sinus membrane during a maxillary sinus augmentation procedure, thereby increasing the success rate of an implant procedure and patient satisfaction.

### BACKGROUND ART

A dental implant is a substitute that replaces the original human tissue when it is lost. However, dentistry defines an implant as a series of procedures to implant a man-made tooth.

In other words, an implant refers to a series of dental procedures including implanting a fixture that is a tooth root made of titanium, which is not rejected by the human body, into the alveolar bone where a tooth has fallen out, to replace a lost tooth root, and then fixing an artificial tooth to restore the function of the tooth.

In the case of general prosthetics or dentures, the surrounding teeth and bones become damaged over time, but implants do not damage the surrounding tooth tissues, and have the same function and shape as natural teeth without developing cavities, so the implants have the advantage of being able to be used semi-permanently.

An artificial tooth procedure (also called an implant or an implant procedure) is completed by making a screw hole, by using a drill, in the alveolar bone at the location where a fixture is to be implanted, implanting the fixture into the screw hole to osseointegrate with the bone to create an artificial tooth root, coupling an abutment to the fixture, and then placing a crown as an artificial tooth that is the final prosthesis, on the abutment.

These implants not only restore a single missing tooth, but also improve the function of dentures in patients with partial or complete edentulism, improve the aesthetic aspect of dental prosthesis restoration, and further help to disperse excessive stress applied to the surrounding supporting bone tissues and stabilize the teeth row.

Meanwhile, there is a procedure called a maxillary sinus augmentation procedure among implant procedures. The maxillary sinus augmentation procedure refers to a procedure of pushing up the maxillary sinus floor, i.e., the maxillary sinus membrane, when the bone height is insufficient during a maxillary implant procedure. For reference, the maxillary sinus refers to a cave next to the nose, i.e., a space between the lower side of an eye and the upper teeth. If pus accumulates here, it becomes sinusitis. Augmentation means lifting.

When performing this type of a maxillary sinus augmentation procedure, after the maxillary sinus membrane is augmented, a drill hole is formed for implanting a fixture. When the diameter of this drill hole is narrow, a second drilling operation must be performed to expand the diameter of the hole.

However, when attempting to secondarily expand the diameter of the drill hole, the drilling work should be performed while preventing the maxillary sinus membrane from being pierced or torn, or in other words, while preventing damage to the maxillary sinus membrane. To do so, it is expected that an appropriate drill tool is used. However, as such a drill tool has not been proposed yet, the development of technology for a new concept of a drill tool for maxillary sinus augmentation is required.

### DESCRIPTION OF EMBODIMENTS

### TECHNICAL PROBLEM

Provided is a drill tool for maxillary sinus augmentation that may easily expand the diameter of a drill hole while preventing damage to the maxillary sinus membrane when a maxillary sinus augmentation procedure is performed, thereby increasing the success rate of an implant procedure and patient satisfaction.

### ADVANTAGEOUS EFFECTS OF DISCLOSURE

According to the present inventive concept, the diameter of a drill hole may be easily expanded while preventing damage to a maxillary sinus membrane when a maxillary sinus augmentation procedure is performed, thereby increasing the success rate of an implant procedure and patient satisfaction

### BRIEF DESCRIPTION OF THE DRAWINGS

- Fig. 1: is a perspective view of a drill tool for maxillary sinus augmentation according to an embodiment of the present inventive concept;
- Fig. 2: is an exploded perspective view of Fig. 1;
- Figs. 3 to 5: are views showing an implant drill at different angles;
- Fig. 6: is a perspective view of a drill housing;
- Fig. 7: is a perspective view of a lower surface of Fig. 6;
- Figs. 8 to 10: are views showing a detailed structure of a region A of Fig. 5 at various angles;
- Figs. 11 to 13: are step-by step usage status views of a drill tool for maxillary sinus augmentation according to an embodiment of the present inventive concept.

### BEST MODE

According to an aspect of the present inventive concept, there is provided a drill tool for maxillary sinus augmentation including an implant drill including a drill hole diameter expansion edge, whereby damage to a membrane of a maxillary sinus is prevented and a diameter of a drill hole is expanded when a maxillary sinus augmentation procedure is performed; and a drill housing that is detachably coupled to the implant drill and guides a drilling operation by the implant drill.

The drill hole diameter expansion edge may include a forward-direction cutting edge that rotates in a forward direction during rotation of the implant drill and cuts a corresponding portion to expand a diameter of the drill hole.

The forward-direction cutting edge may be a forward-direction round cutting edge with a round end portion.

The drill hole diameter expansion edge further may include a material moving guide that is arranged adjacent to the forward-direction cutting edge and guides bone chips generated during the drilling operation or a bone grafting material input through the drill housing to be pushed upward in a direction toward the maxillary sinus.

The material moving guide may be a reverse-direction material flute edge that is processed in a reverse direction to a direction of the forward-direction cutting edge, whereby the reverse-direction material flute edge prevents the bone chips or the bone grafting material from being accumulated in the forward-direction cutting edge or around the forward-direction cutting edge during the drilling operation.

In the implant drill, the drill hole diameter expansion edge may be disposed in an end portion area, and the implant drill may further includes a drill shaft in which a device mounting portion is formed at a side opposite to the drill hole diameter expansion edge.

The implant drill further may include a housing attaching/detaching coupling portion provided in the drill shaft area at a position where the drill hole diameter expansion edge ends, and forming a place where the drill housing is detachably coupled.

the housing attaching/detaching coupling portion may include: a housing supporting boss formed at a position where the drill hole diameter expansion edge ends to be larger than a cross-sectional area of the drill shaft and partially supporting the drill housing; and a stopper flange having a diameter greater than the housing supporting boss, formed in an end portion of the housing supporting boss, and forming a coupling line along which the drill housing is coupled.

A detachable protrusion may be provided on one of the housing supporting boss and the drill housing of the implant drill, and a detachable protrusion groove to which the detachable protrusion is detachably coupled may be formed on the other one of the housing supporting boss and the drill housing of the implant drill.

The detachable protrusion may be formed to protrude radially outward from the housing supporting boss of the implant drill, and the detachable protrusion groove may be formed to be recessed radially inward from an inner wall of the drill housing.

The drill housing may include a hollow housing body having one end portion area that is supported in contact with an area of the housing supporting boss of the housing attaching/detaching coupling portion and the detachable protrusion groove formed on one side of the inner wall.

A plurality of elasticity providing cutting grooves that provide elasticity may be formed in an end portion area of the hollow housing body in which the detachable protrusion groove is positioned.

The plurality of elasticity providing cutting grooves may be arranged at equal intervals along a circumferential direction of the hollow housing body.

At least one through-hole may be formed in a wall surface of the hollow housing body.

The through-hole may be provided as a slot, and a plurality of through-holes may be arranged at equal intervals along the circumferential direction of the hollow housing body.

### MODE OF DISCLOSURE

The attached drawings for illustrating preferred embodiments of the present inventive concept are referred to in order to gain a sufficient understanding of the present inventive concept, the merits thereof, and the objectives accomplished by the implementation of the present inventive concept.

Hereinafter, the present inventive concept will be described in detail by explaining preferred embodiments of the present inventive concept with reference to the attached drawings. Like reference numerals in the drawings denote like elements.

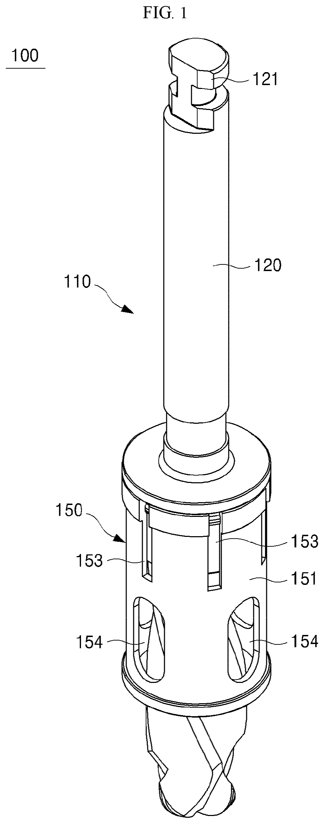

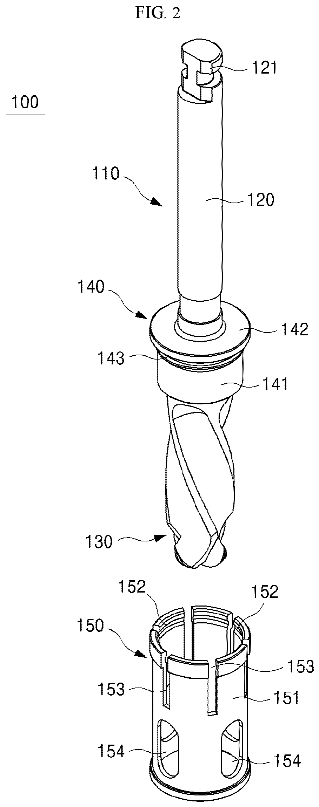

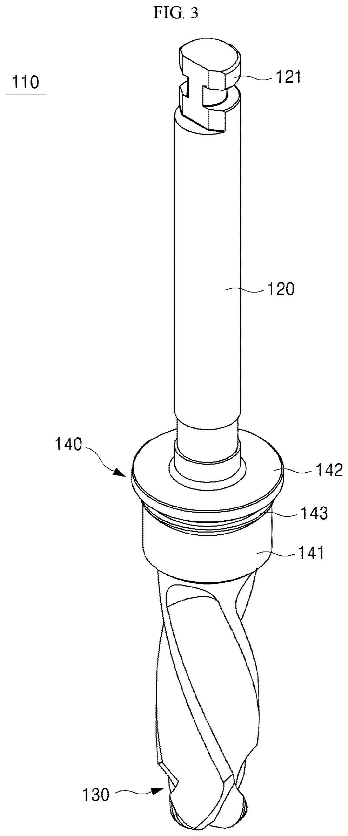

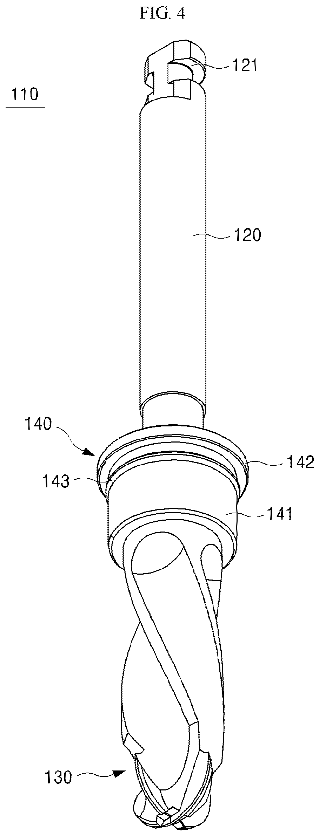

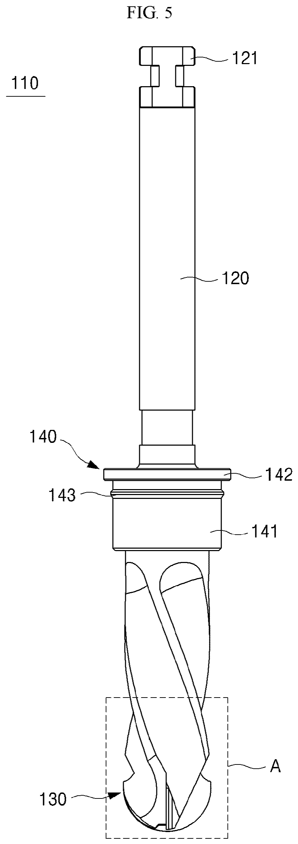

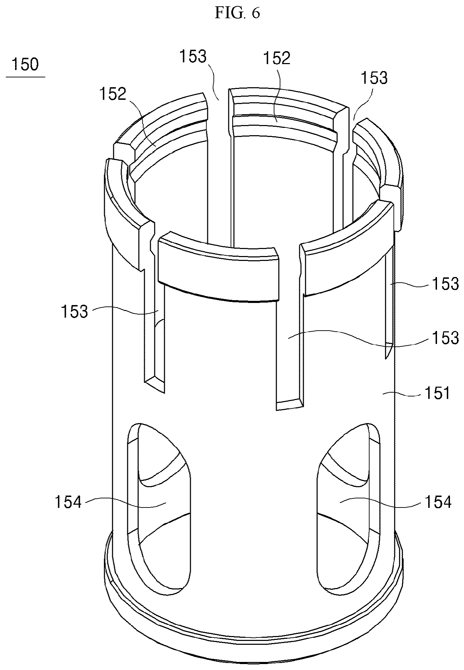

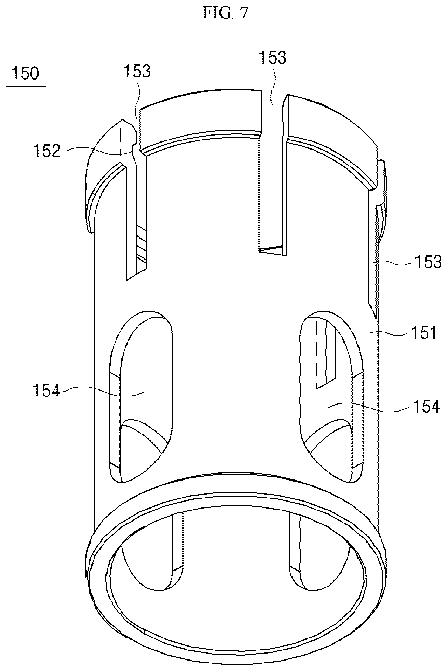

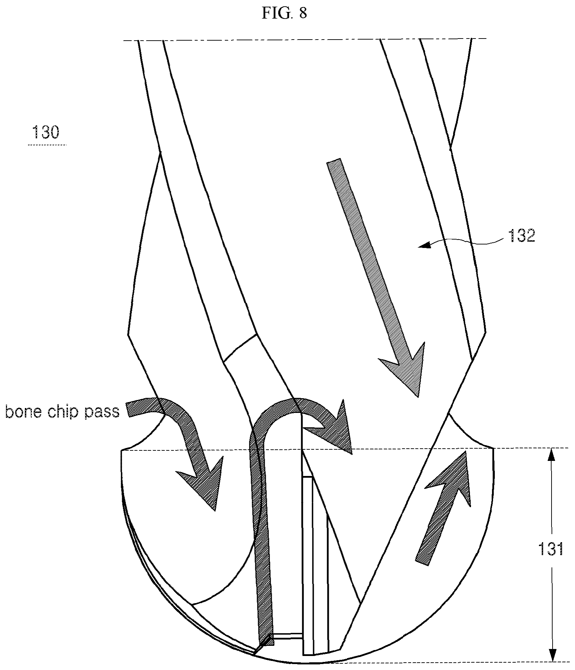

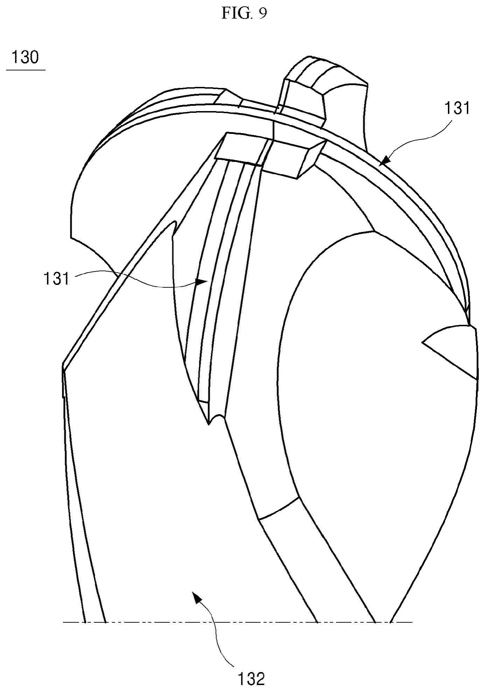

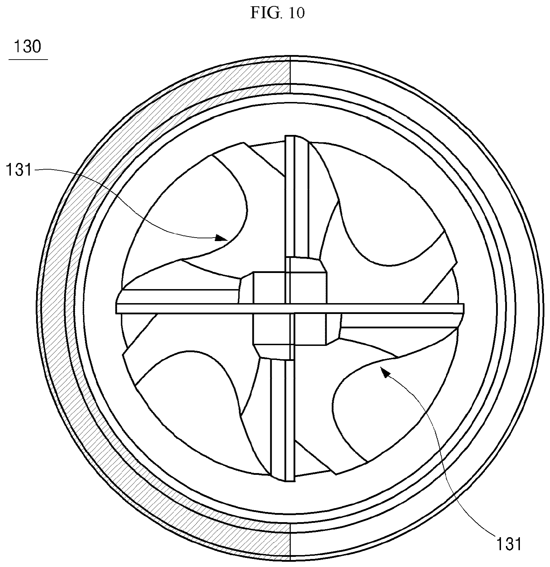

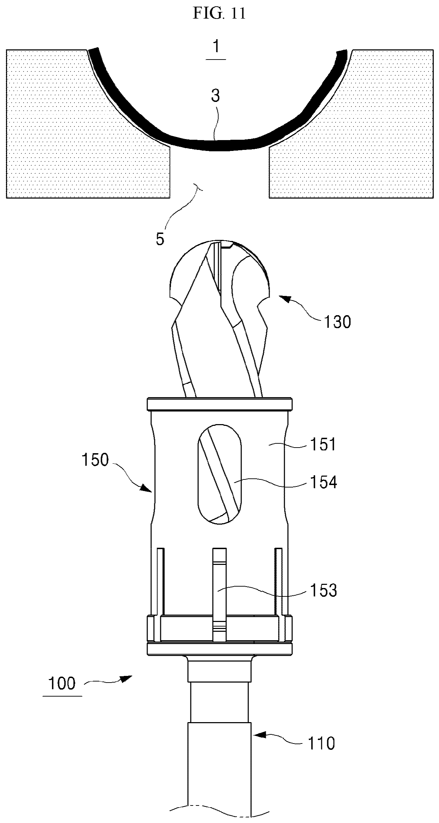

FIG. 1 is a perspective view of a drill tool for maxillary sinus augmentation according to an embodiment of the present inventive concept. FIG. 2 is an exploded perspective view of FIG. 1. FIGS. 3 to 5 are views showing an implant drill at different angles. FIG. 6 is a perspective view of a drill housing. FIG. 7 is a perspective view of a lower surface of FIG. 6. FIGS. 8 to 10 are views showing a detailed structure of a region A of FIG. 5 at various angles. FIGS. 11 to 13 are step-by step usage status views of a drill tool for maxillary sinus augmentation according to an embodiment of the present inventive concept.

Referring to these drawings, a drill tool 100 for maxillary sinus augmentation according to the present embodiment may easily expand the diameter of a drill hole 5 while preventing damage to a membrane 3 of a maxillary sinus 1 during augmentation of the maxillary sinus 1, thereby increasing the success rate of an implant procedure and patient satisfaction, and may include 2 parts that can be mutually disassembled and assembled, that is, an implant drill 110 and a drill housing 150.

The implant drill 110 and the drill housing 150 are independent structures, and in a connected and assembled state as shown in FIG. 1, are arranged around the upper jaw as shown in FIGS. 11 to 13 and rotated by a separate power drill apparatus (not shown) to perform a drilling operation, that is, a drilling operation to expand the diameter of the drill hole 5.

First, the implant drill 110 is described. The implant drill 110 is a substantial means and structure to expand the diameter of the drill hole 5. When the drill hole 5 into which a fixture is to be placed is formed in the maxilla as shown in FIG. 11, the implant drill 110 may be used to expand the diameter of the drill hole 5.

While coupled to the drill housing 150, the implant drill 110 rotates together with the drill housing 150 to perform a drilling operation.

The implant drill 110 may include a drill shaft 120 and a drill hole diameter expansion edge 130 provided in one side end area of the drill shaft 120.

The drill shaft 120 has a long rod type structure, and the drill hole diameter expansion edge 130 is provided in one end area thereof.

A device mounting portion 121 is provided at an end portion of the drill shaft 120 opposite to the drill hole diameter expansion edge 130. The device mounting portion 121 may be used to mount the drill tool 100 for maxillary sinus augmentation according to the present embodiment on the separate power drill apparatus.

A housing attaching/detaching coupling portion 140 is provided in the central area of the drill shaft 120. The housing attaching/detaching coupling portion 140 is provided in an area of the drill shaft 120 at a position where the drill hole diameter expansion edge 130 ends, and forms a place where the drill housing 150 is detachably coupled.

The housing attaching/detaching coupling portion 140 may include a housing supporting boss 141 and a stopper flange 142.

The housing supporting boss 141 is a portion formed at a position where the drill hole diameter expansion edge 130 ends and formed to be greater than the cross-sectional area of the drill shaft 120. The housing supporting boss 141 supports an area of a hollow housing body 151 of the drill housing 150 where an elasticity providing cutting groove 153 is positioned.

The stopper flange 142 is formed at an end of the housing supporting boss 141. The stopper flange 142 has a diameter greater than that of the housing supporting boss 141. Accordingly, while an end portion of the drill housing 150 is supported on the stopper flange 142, the drill housing 150 may be coupled to the housing attaching/detaching coupling portion 140.

A detachable protrusion 143 and a detachable protrusion groove 152 are formed such that the drill housing 150 is detachably coupled to the implant drill 110.

The detachable protrusion 143 is provided at one of the housing supporting boss 141 of the implant drill 110 and the drill housing 150, and the detachable protrusion groove 152 is provided at the other of the housing supporting boss 141 of the implant drill 110 and the drill housing 150.

In the case of the present embodiment, the detachable protrusion 143 is formed to protrude radially outward from the housing supporting boss 141 of the implant drill 110, and the detachable protrusion groove 152 is formed to be recessed radially inward from an inner wall of the drill housing 150. Accordingly, when the drill housing 150 is inserted around the implant drill 110, the detachable protrusion groove 152 of the drill housing 150 is inserted into the detachable protrusion 143 provided on the housing supporting boss 141 of the implant drill 110 so as to be easily coupled to the drill housing 150.

Unlike the drawings, the detachable protrusion 143 may be formed in the drill housing 150, and the detachable protrusion groove 152 may be formed in the implant drill 110. All of these matters should be construed as falling within the scope of the present inventive concept.

Meanwhile, the drill hole diameter expansion edge 130 is a portion formed in an end portion area of the drill shaft 120 and is a device to substantially perform a drilling operation. In other words, the drill hole diameter expansion edge 130 may prevent the membrane 3 of the maxillary sinus 1 from being damaged when the augmentation procedure of the maxillary sinus 1 is performed and also expand the diameter of the drill hole 5.

The drill hole diameter expansion edge 130 may include a forward-direction cutting edge 131 and a material moving guide 132.

The forward-direction cutting edge 131 is a device that cuts a corresponding portion to expand the diameter of the drill hole 5 while rotating in a forward direction during rotation of the implant drill 110.

In the present embodiment, the forward-direction cutting edge 131 is a forward-direction round cutting edge 131 having a round end portion, and a plurality of forward-direction cutting edges are provided as the forward-direction cutting edge 131 in the end portion area of the drill shaft 120. The forward-direction round cutting edges 131 are concentrically arranged with respect to a center point.

The material moving guide 132 is arranged adjacent to the forward-direction cutting edge 131, and guides bone chips generated during a drilling operation or a bone grafting material that can be input through the drill housing 150 to be pushed upward in a direction toward the maxillary sinus 1.

In the present embodiment, a reverse-direction material flute edge 132 that is processed in a reverse direction to the direction of the forward-direction cutting edge 131 is applied as the material moving guide 132. The reverse-direction material flute edge 132 is arranged in a twisted type around the forward-direction cutting edge 131.

As such, as the drill hole diameter expansion edge 130 is configured as the forward-direction round cutting edge 131 and the reverse-direction material flute edge 132, during a drilling operation in FIGS. 11 to 13, the bone chips or the bone grafting material may be prevented from being accumulated in the forward-direction round cutting edge 131 or around the forward-direction round cutting edge 131.

Next, the drill housing 150 is detachably coupled to the implant drill 110, and is a device that guides a drilling operation by the implant drill 110.

In particular, the drill housing 150 is used as a device for collecting bone chips generated during a drilling operation and also for injecting the bone grafting material.

The drill housing 150 includes the hollow housing body 151 having one end portion area that is supported in contact with an area of the housing supporting boss 141 of the housing attaching/detaching coupling portion 140 and the detachable protrusion groove 152 that is formed on one side of the inner wall. As the hollow housing body 151 has a hollow pipe structure, the drill housing 150 is easily attached/detached.

A plurality of elasticity providing cutting grooves 153 for providing elasticity are formed in an end portion area of the housing body 151 with the hollow inside where the detachable protrusion groove 152 is located.

The plurality of elasticity providing cutting grooves 153 are arranged at equal intervals along the circumferential direction of the hollow housing body 151. As such, as the plurality of elasticity providing cutting grooves 153 are formed in an end portion area of the hollow housing body 151, unlike the integrated structure, the end portion area of the hollow housing body 151 may retain a certain elastic force.

Accordingly, when the drill housing 150 is inserted around the implant drill 110, as the area of the elasticity providing cutting grooves 153 slightly opens and then elastically closes, the detachable protrusion groove 152 may be coupled to the detachable protrusion 143 of the implant drill 110, and thus the drill housing 150 is prevented from being detached inadvertently.

In addition, a through-hole 154 is formed in a wall surface of the hollow housing body 151. In the present embodiment, the through-hole 154 is provided as a slot, and a plurality of through-holes may be arranged at equal intervals along the circumferential direction of the hollow housing body 151. The through-hole 154 forms a place where the bone grafting material is input.

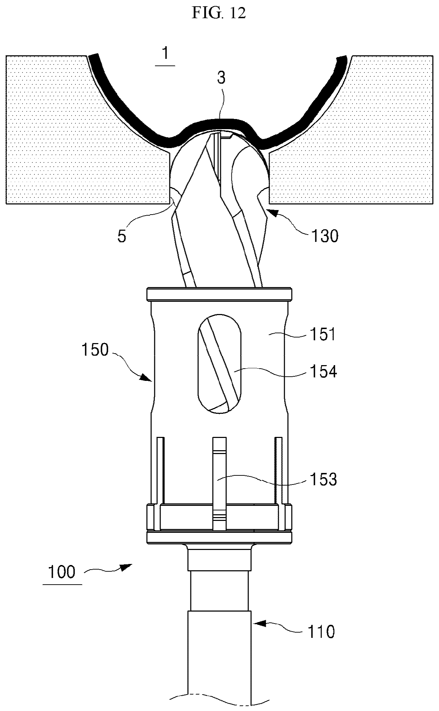

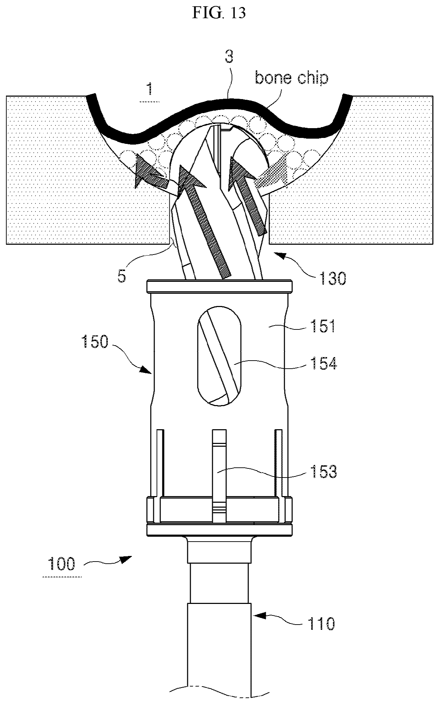

According to the above configuration, when the augmentation procedure of the maxillary sinus 1 is performed, if there is a need to expand the diameter of the drill hole 5 that is previously punched and small, the drill tool 100 for maxillary sinus augmentation according to the present embodiment is positioned as illustrated in FIG. 11, and inserted into the drill hole 5 as illustrated in FIGS. 12 and 13 so as to perform a drilling operation. Then, the bone chips or the bone grafting material may be prevented from being accumulated in the forward-direction round cutting edge 131 or around the forward-direction round cutting edge 131 and also may be pushed upward toward the maxillary sinus 1, and the membrane 3 of the maxillary sinus 1 may be prevented from being damaged and the diameter of the drill hole 5 may be expanded.

According to the present embodiment that operates with the structure described above, the diameter of the drill hole 5 may be easily expanded while preventing damage to the membrane 3 of the maxillary sinus 1 when the augmentation procedure of the maxillary sinus 1 is performed, thereby increasing the success rate of an implant procedure and patient satisfaction.

While this inventive concept has been particularly shown and described with reference to preferred embodiments thereof, it will be understood by those of ordinary skill in the art that various changes in form and details may be made therein without departing from the spirit and scope of the inventive concept as defined by the appended claims. Therefore, the scope of the inventive concept is defined not by the detailed description of the inventive concept but by the appended claims, and all differences within the scope will be construed as being included in the present inventive concept.

### INDUSTRIAL APPLICABILITY

The present inventive concept may be used for a dental procedure.

## Claims

1. A drill tool for maxillary sinus augmentation comprising: an implant drill including a drill hole diameter expansion edge, whereby damage to a membrane of a maxillary sinus is prevented and a diameter of a drill hole is expanded when a maxillary sinus augmentation procedure is performed; and
a drill housing that is detachably coupled to the implant drill and guides a drilling operation by the implant drill.

2. The drill tool for maxillary sinus augmentation of claim 1, wherein the drill hole diameter expansion edge includes a forward-direction cutting edge that rotates in a forward direction during rotation of the implant drill and cuts a corresponding portion to expand a diameter of the drill hole.

3. The drill tool for maxillary sinus augmentation of claim 2, wherein the forward-direction cutting edge is a forward-direction round cutting edge with a round end portion.

4. The drill tool for maxillary sinus augmentation of claim 2, wherein the drill hole diameter expansion edge further includes a material moving guide that is arranged adjacent to the forward-direction cutting edge and guides bone chips generated during the drilling operation or a bone grafting material input through the drill housing to be pushed upward in a direction toward the maxillary sinus.

5. The drill tool for maxillary sinus augmentation of claim 4, wherein the material moving guide is a reverse-direction material flute edge that is processed in a reverse direction to a direction of the forward-direction cutting edge, whereby the reverse-direction material flute edge prevents the bone chips or the bone grafting material from being accumulated in the forward-direction cutting edge or around the forward-direction cutting edge during the drilling operation.

6. The drill tool for maxillary sinus augmentation of claim 1, wherein the implant drill further comprises the drill hole diameter expansion edge is disposed in an end portion area, the implant drill further comprising a drill shaft in which a device mounting portion is formed at a side opposite to the drill hole diameter expansion edge.

7. The drill tool for maxillary sinus augmentation of claim 6, wherein the implant drill further comprises a housing attaching/detaching coupling portion provided in the drill shaft area at a position where the drill hole diameter expansion edge ends, and forming a place where the drill housing is detachably coupled.

8. The drill tool for maxillary sinus augmentation of claim 7, wherein the housing attaching/detaching coupling portion comprises:
a housing supporting boss formed at a position where the drill hole diameter expansion edge ends to be larger than a cross-sectional area of the drill shaft and partially supporting the drill housing; and
a stopper flange having a diameter greater than the housing supporting boss, formed in an end portion of the housing supporting boss, and forming a coupling line along which the drill housing is coupled.

9. The drill tool for maxillary sinus augmentation of claim 8, wherein a detachable protrusion is provided on one of the housing supporting boss and the drill housing of the implant drill, and a detachable protrusion groove to which the detachable protrusion is detachably coupled is formed on the other one of the housing supporting boss and the drill housing of the implant drill.

10. The drill tool for maxillary sinus augmentation of claim 9, wherein the detachable protrusion is formed to protrude radially outward from the housing supporting boss of the implant drill, and the detachable protrusion groove is formed to be recessed radially inward from an inner wall of the drill housing.

11. The drill tool for maxillary sinus augmentation of claim 10, wherein the drill housing comprises a hollow housing body having one end portion area that is supported in contact with an area of the housing supporting boss of the housing attaching/detaching coupling portion and the detachable protrusion groove formed on one side of the inner wall.

12. The drill tool for maxillary sinus augmentation of claim 11, wherein a plurality of elasticity providing cutting grooves that provide elasticity are formed in an end portion area of the hollow housing body in which the detachable protrusion groove is positioned.

13. The drill tool for maxillary sinus augmentation of claim 12, wherein the plurality of elasticity providing cutting grooves are arranged at equal intervals along a circumferential direction of the hollow housing body.

14. The drill tool for maxillary sinus augmentation of claim 11, wherein at least one through-hole is formed in a wall surface of the hollow housing body.

15. The drill tool for maxillary sinus augmentation of claim 14, wherein the through-hole is provided as a slot, and a plurality of through-holes are arranged at equal intervals along the circumferential direction of the hollow housing body.
